(19) Europäisches Patentamt
European Patent Office
Office européen des brevets

(11) Veröffentlichungsnummer: **0 271 804 B1**

(12) # EUROPÄISCHE PATENTSCHRIFT

(45) Veröffentlichungstag der Patentschrift: **18.03.92**

(51) Int. Cl.$^5$: **C07D 471/04**, C07D 239/30, //(C07D471/04,239:20,221:00)

(21) Anmeldenummer: **87118125.1**

(22) Anmeldetag: **08.12.87**

(54) **Cyan-trichlor-pyrido-pyrimidin.**

(30) Priorität: **19.12.86 DE 3643456**

(43) Veröffentlichungstag der Anmeldung:
**22.06.88 Patentblatt 88/25**

(45) Bekanntmachung des Hinweises auf die Patenterteilung:
**18.03.92 Patentblatt 92/12**

(84) Benannte Vertragsstaaten:
**CH DE FR GB LI**

(56) Entgegenhaltungen:
**DE-A- 2 310 334**

**HEINZ BECKER, "Einführung in die Elektronentheorie organisch-chemischer Reaktionen", 1961, VEB DEUTSCHER VERLAG DER WISSENSCHAFTEN, Berlin, Seiten 387-399**

**BEILSTEINS HANDBUCH DER ORGANISCHEN CHEMIE, 4. Auflage, 3. und 4. Ergänzungswerk, Band 22/2, Seiten 1175, 1176**

(73) Patentinhaber: **BAYER AG**

**W-5090 Leverkusen 1 Bayerwerk(DE)**

(72) Erfinder: **Beck, Gunther, Dr.**
**Am Mittelberg 19**
**W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 271 804 B1

## Beschreibung

Gegenstand der vorliegenden Erfindung ist das neue Cyan-trichlor-pyrido-pyrimidin der Formel

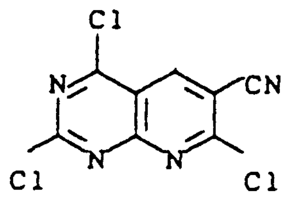

(I),

sowie ein Verfahren zu seiner Herstellung.

Das Verfahren ist dadurch gekennzeichnet, daß man das Malodinitril-Kondensat der Formel

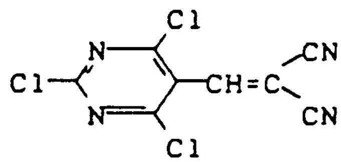

(II),

gegebenenfalls in Gegenwart von Friedel-Crafts-Verbindungen, auf höhere Temperaturen erhitzt.

Im allgemeinen wird im Temperaturbereich von etwa 100 bis 300 ° C, vorzugsweise zwischen 150 und 250 ° C, gearbeitet.

Geeignete Friedel-Crafts-Verbindungen sind z.B. beschrieben in "Friedel Crafts and Related Reactions", Volume I, Seite 201.

Beispielsweise seien aufgeführt: $AlCl_3$, $AlBr_3$, $BeCl_2$, $CdCl_2$, $ZnCl_2$, $BF_3$, $BCl_3$, $BBr_3$, $GaCl_3$, $TiCl_4$, $TiBr_4$, $ZrCl_4$, $SnCl_4$, $SnBr_4$, $SbCl_5$, $SbCl_3$, $FeCl_3$, $UCl_4$.

Besonders bevorzugt ist $AlCl_3$.

Im allgemeinen setzt man die Friedel-Crafts-Verbindungen in einer Menge von 0,01 bis 20 Gewichtsprozent, vorzugsweise 0,1 bis 10 Gewichtsprozent bezogen auf (II) ein.

Der Reaktionsablauf kann durch das folgende Formelschema wiedergegeben werden:

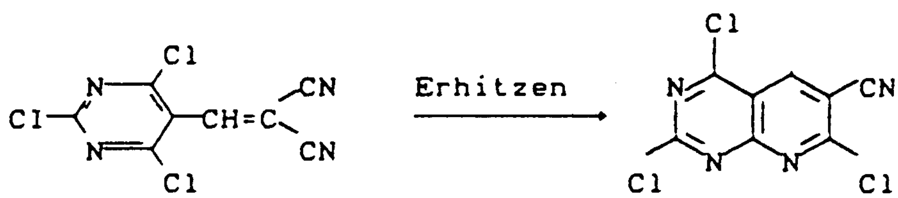


(II) (I)

Das als Ausgangsstoff verwendete Malodinitril-Kondensat der Formel (II) ist neu und wird folgendermaßen hergestellt:

Ausgehend von dem in bekannter Weise (DE-A 2 310 334) aus Barbitursäure, N,N-Dimethylformamid und Phosphorylchlorid zugänglichen bekannten 2,4,6-Trichlor-pyrimidin-5-aldehyd (III) wird mit Malodinitril (IV) gemäß der Reaktionsgleichung

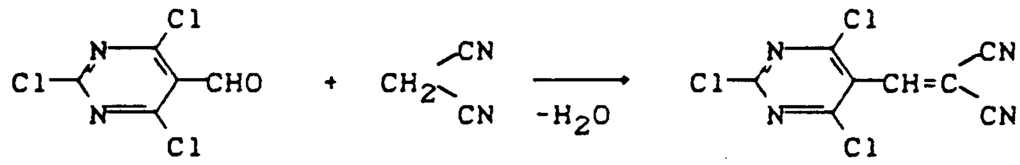

(III) (IV) (II)

in an sich bekannter Weise eine sogenannte Knoevenagel-Kondensation durchgeführt.

Knoevenagel-Kondensationen sind z.B. beschrieben in "Organikum", 13. Auflage, Seite 508-510. Nähere Einzelheiten sind dem Versuchsteil zu entnehmen.

Zur Durchführung des erfindungsgemäßen Verfahrens wird die Ausgangsverbindung der Formel (II), gegebenenfalls mit bevorzugt 0,1 bis 10 Gew.-% einer Friedel-Crafts-Verbindung, bevorzugt Aluminiumchlorid, vermischt und so lange auf den angegebenen Temperaturbereich, vorzugsweise auf 150 bis 250 °C, erhitzt, bis das erfindungsgemäße Cyan-trichlorpyrido-pyrimidin der Formel (I) gebildet ist. Zweckmäßig arbeitet man unter Feuchtigkeitsausschluß. Der Zeitbedarf hängt einmal davon ab, ob mit oder ohne Zusatz von Friedel-Crafts-Katalysatoren gearbeitet wird, zum anderen naturgemäß von Art und Menge der Friedel-Crafts-Verbindung sowie von der Reaktions-temperatur. Im allgemeinen liegt er in der Größenordnung von 0,1 bis 30 Stunden. Katalysator-Zusatz verringert den Zeitbedarf.

Selbstverständlich kann man auch kontinuierlich arbeiten.

Die Reindarstellung des Umlagerungsprodukts (I) kann nach üblichen Methoden der organischen Chemie erfolgen, beispielsweise durch Vakuumsublimation. Die Ausbeuten an sublimiertem, reinweißem Produkt betragen zwischen 75 und 95 % der Theorie.

Das Cyan-trichlor-pyrido-pyrimidin (I) ist als Reaktivkomponente für die Herstellung von Reaktivfarbstoffen geeignet. Hierzu wird es z.B. in üblicher Weise mit äquimolaren Mengen eines aminogruppenhaltigen Azofarbstoffs umgesetzt. Dabei erhält man Reaktivfarbstoffe, die auf Baumwolle kräftige klare Färbungen mit guter Wasch- und Lichtechtheit liefern.

Beispiel 1

30,20 g Malodinitril-Kondensat (II) wurden in einem Glasschiffchen in einer stufenlos elektrisch beheizbaren Trockenpistole, an deren Hahnstutzen bei geöffnetem Hahn eine Gummiblase ("Orsat" -Blase) befestigt war, 5 Stunden auf 190 bis 195 °C erhitzt. Die IR-spektroskopische Analyse des erkalteten Reaktionsprodukts zeigte, daß kein Ausgangsprodukt mehr vorhanden war. Durch anschließende Vakuum-Sublimation bei 180 °C/0,1 mb erhielt man ein reinweißes Sublimat (I) vom Schmelzpunkt 184 °C. Sublimationsrückstand: 2,09 g. Damit ergibt sich eine Ausbeute von 93,1 % der Theorie an Cyan-trichlorpyrido-pyrimidin der Formel (I). I.R. (KBr), $\nu$ = 3.063, 2.236, 1.595, 1.561, 1.524, 1.421, 1.397, 1.329, 1.228, 1.144, 1.071, 1.064, 948, 863, 805 $cm^{-1}$.

Herstellung des Malodinitril-Kondensats (II):

a) In 6 Liter Acetonitril wurden 1 kg (4,73 Mol) 2,4,6-Trichlor-pyrimidin-5-aldehyd (III), 350 g (5,3 Mol) Malodinitril (IV) und 420 g (4,7 Mol) 3-Amino-propansäure eingetragen und die Reaktionsmischung bei Raumtemperatur unter Feuchtigkeitsausschluß etwa 20 Stunden kräftig gerührt. Danach zeigte die dünnschichtchromatographische Analyse, daß kein Aldehyd (III) mehr vorhanden war. Das Reaktionsgemisch wurde anschließend in 30 Liter Wasser verrührt, dann wurde abfiltriert, mit Wasser gewaschen und bei Raumtemperatur getrocknet. Ausbeute an Malodinitril-Kondensat (II) 933 g (76 % der Theorie). Die Verbindung kann aus Toluol umkristallisiert oder bei z.B. 120 °C/0,1 mb sublimiert werden. Schmelzpunkt 160 °C.

I.R. (KBr) : $\nu$ = 3.030, 2.240, 1.612, 1.544, 1.492, 1.356, 1.341, 1.302, 1.227, 1.160, 1.145, 911, 890, 829, 790 $cm^{-1}$.

b) Eine Lösung von 21, 15 g (0,1 Mol) 2,4,6-Trichlor-pyrimidin-5-aldehyd (III) und 13,2 g (0,2 Mol) Malodinitril in 100 ml Benzol wurde mit 1,0 g Ammoniumacetat und 1,2 g Eisessig versetzt und 4 Stunden am Wasserabscheider under Rückfluß erhitzt. Danach wurde heiß von wenig (unter 1 g) Ungelöstem abfiltriert und das Filtrat im Rotationsverdampfer eingeengt. Der auf Ton nachgetrocknete Rotavapor-Rückstand wurde in der Trockenpistole etwa 2 Stunden bei 85 °C/0,1 mbar gehalten. Hierbei

konnten 4,85 g unumgesetzter 2,4,6-Trichlor-pyrimidin-5-aldehyd als Sublimat zurückgewonnen werden. Als Rückstand hinterblieben 15,57 g (60 % Ausbeute bzw. 77,9 % Ausbeute, bezogen auf umgesetzten 2,4,6-Trichlor-pyrimidin-5-aldehyd) Malodinitril-Kondensat (II), in allen Eigenschaften identisch mit dem unter a) gewonnenen Produkt.

Beispiel 2

In einer Apparatur analog Beispiel 1 wurden 12,78 g Malodinitril-Kondensat (II) mit 0,2 g $AlCl_3$ versetzt und eine Stunde auf 160 bis 165 °C erhitzt. Die IR-spektroskopische Analyse des erkalteten Reaktionsprodukts zeigte, daß kein Ausgangsprodukt mehr vorhanden war und daß sich das Pyrido-pyrimidin (I) gebildet hatte.

Beispiel 3

In einer Apparatur analog Beispiel 1 wurden 18,60 g Malodinitril-Kondensat (II) mit 0,3 g $AlCl_3$ versetzt und 70 Minuten auf etwa 195 °C erhitzt. Anschließend wurde bei 180 °C/0,1 mb sublimiert. Das reinweiße Sublimat war identisch mit dem des Beispiels 1. Sublimationsrückstand: 4,63 g, entsprechend einer Ausbeute an Cyan-trichlor-pyrido-pyrimidin (I) von 75 %.

Beispiel 4

0,029 Mol des Farbstoffs der Formel

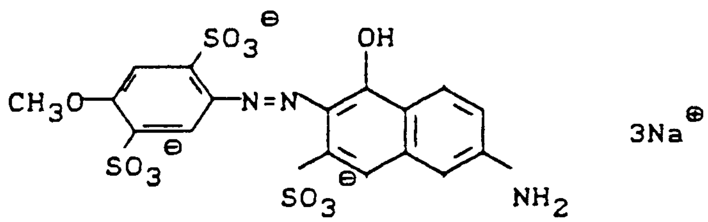

werden in 250 ml Wasser gerührt. Man streut 7,5 g (0,029 Mol) feinpulverisiertes Pyrido-pyrimidin der Formel

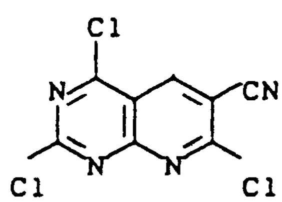

ein, erwärmt auf 40 °C und neutralisiert die freiwerdende Salzsäure durch Zutropfen von verdünnter Sodalösung.

Nach beendeter Acylierung wird das angefallene Kondensationsprodukt abgesaugt, getrocknet und gemahlen. Man erhält ein orangefarbenes, in Wasser lösliches Farbstoffpulver. Der Farbstoff entspricht wahrscheinlich der Formel

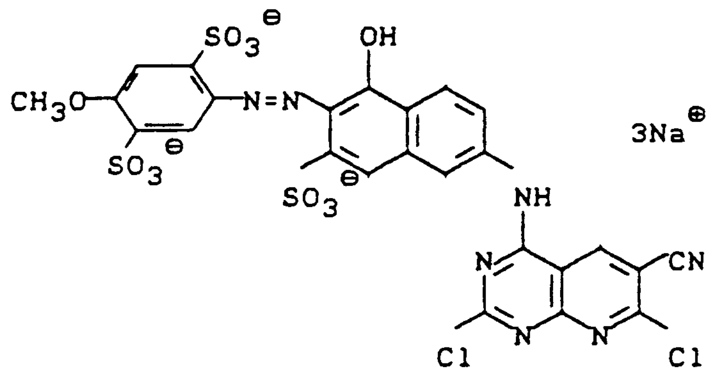

Beispiel 5

0,029 Mol des Farbstoffs der Formel

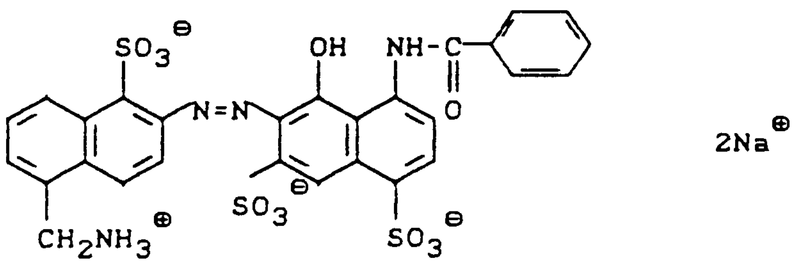

werden in 400 ml Wasser verrührt. Bei Raumtemperatur streut man 7,5 g (0,029 Mol) fein pulverisiertes Pyrido-pyrimidin der Formel

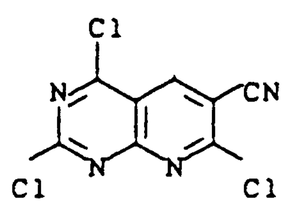

ein und hält durch Eintropfen von 6 %iger Lithiumhydroxidlösung den pH zwischen 8,5 und 8,8. Nach beendeter Acylierung wird der Farbstoff durch Zugabe von 200 ml gesättigter Kochsalzlösung ausgesalzen. Nach dem Absaugen, Trocknen und Mahlen erhält man ein rotes Farbstoffpulver, das sich leicht in Wasser löst. Der Farbstoff entspricht wahrscheinlich der Formel

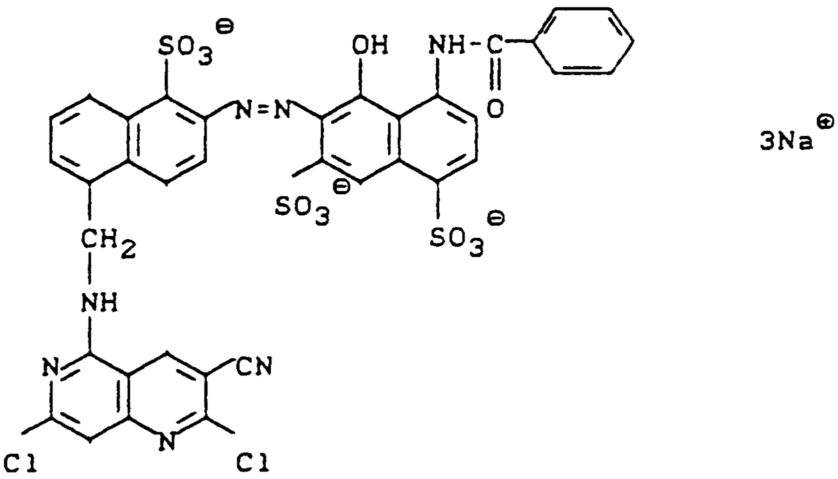

Färbevorschrift

2 Teile des gemäß Beispiel 5 erhältlichen Farbstoffs werden in 100 Teilen Wasser gelöst.

Die Lösung gibt man zu 1.900 Teilen kaltem Wasser, fügt 60 Teile Natriumchlorid zu und geht mit 100 Teilen eines Baumwollstranges in dieses Färbebad ein.

Man steigert die Temperatur auf 70 bis 80 ° C, wobei nach 30 Minuten 40 Teile kalzinierte Soda und nochmals 60 Teile Natriumchlorid zugegeben werden. Man hält die Temperatur 30 Minuten auf 70 bis 80 ° C, spült und seift dann die Färbung während 15 Minuten in einer 0,3 %igen kochenden Lösung eines ionenfreien Waschmittels, spült und trocknet.

Man erhält eine kräftige blaustichig-rote Färbung mit guter Wasch- und Lichtechtheit.

Verwendet man 2 Teile des nach Beispiel 4 erhältlichen Farbstoffes, so resultiert ein klares rotstichiges Orange auf Baumwolle.

**Patentansprüche**

**1.** Cyan-trichlor-pyrido-pyrimidin der Formel

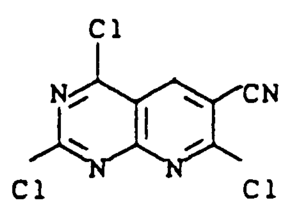

**2.** Verfahren zur Herstellung des Cyan-trichlor-pyrido-pyrimidins des Anspruchs 1, dadurch gekennzeichnet, das man das Malodinitril-Kondensat der Formel

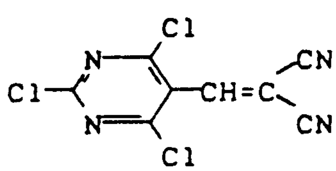

auf Temperaturen von etwa 100 bis 300 ° C, vorzugsweise 150 bis 250 ° C, erhitzt.

**3.** Verfahren nach Anspruch 2, dadurch gekennzeichnet, daß man in Gegenwart von Friedel-Crafts-Katalysatoren arbeitet.

**4.** Malodinitril-Kondensat der Formel

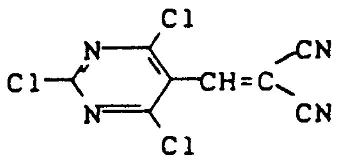

**Claims**

**1.** Cyano-trichloro-pyrido-pyrimidine of the formula

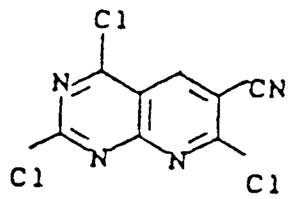

**2.** Process for the preparation of the cyano-trichloro-pyrido-pyrimidine of Claim 1, characterized in that the malodinitrile condensate of the formula

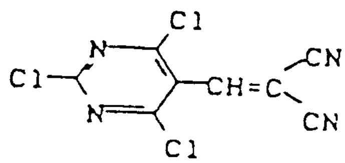

is heated at temperatures of about 100 to 300 ° C, preferably 150 to 250 ° C.

**3.** Process according to Claim 2, characterized in that it is carried out in the presence of Friedel-Crafts catalysts.

**4.** Malodinitrile condensate of the formula

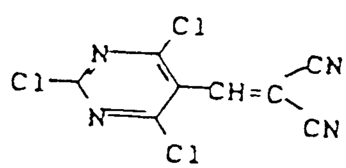

**Revendications**

**1.** Cyanotrichloropyridopyrimidine de formule :

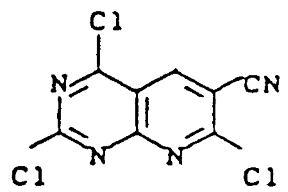

**2.** Procédé pour la fabrication de la cyanotrichloropyridopyrimidine selon la revendication 1, caractérisé en ce que l'on chauffe le condensat de malodinitrile de formule :

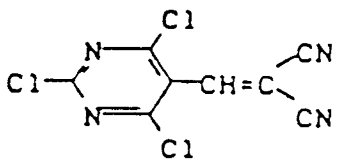

à des températures d'environ 100 à 300 ° C de préférence de 150 à 250 ° C.

**3.** Procédé selon la revendication 2, caractérisé en ce que l'on opère en présence de catalyseurs de Friedel-Crafts.

**4.** Condensat de malodinitrile de formule :